(19) Europäisches Patentamt / European Patent Office / Office européen des brevets

(11) **EP 3 010 413 B1**

(12) **EUROPÄISCHE PATENTSCHRIFT**

(45) Veröffentlichungstag und Bekanntmachung des Hinweises auf die Patenterteilung:
**04.12.2019 Patentblatt 2019/49**

(21) Anmeldenummer: **14730818.3**

(22) Anmeldetag: **06.06.2014**

(51) Int Cl.:
**A61B 5/06** *(2006.01)*   **G01R 33/09** *(2006.01)*

(86) Internationale Anmeldenummer:
**PCT/EP2014/061773**

(87) Internationale Veröffentlichungsnummer:
**WO 2014/202404 (24.12.2014 Gazette 2014/52)**

(54) **PERSONALISIERTES DETEKTIONSSYSTEM ZUR ERFASSUNG MAGNETISCHER OBJEKTE IM MENSCHLICHEN ORGANISMUS**

PERSONALIZED DETECTION SYSTEM FOR DETECTING MAGNETIC OBJECTS IN THE HUMAN ORGANISM

SYSTÈME DE DÉTECTION PERSONNALISÉ POUR DÉTECTER DES OBJETS MAGNÉTIQUES DANS LE CORPS HUMAIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.06.2013 DE 102013211703**

(43) Veröffentlichungstag der Anmeldung:
**27.04.2016 Patentblatt 2016/17**

(73) Patentinhaber: **Evonik Degussa GmbH**
**45128 Essen (DE)**

(72) Erfinder:
• HARTWIG, Benedikt
  **64287 Darmstadt (DE)**
• NIEPOTH, Peter
  **64823 Groß-Umstadt (DE)**
• STILLER, Hans-Joachim
  **verstorben (DE)**
• JUNGINGER, Steffen
  **18258 Bröbberow OT Gross Grenz (DE)**

(74) Vertreter: **Evonik Patent Association**
**c/o Evonik Industries AG**
**IP Management**
**PB 84/339**
**Rodenbacher Chaussee 4**
**63457 Hanau (DE)**

(56) Entgegenhaltungen:
**US-A1- 2007 167 743   US-A1- 2010 322 859**
**US-A1- 2011 275 907   US-A1- 2012 317 064**

• CHAO HU ET AL: "A Cubic 3-Axis Magnetic Sensor Array for Wirelessly Tracking Magnet Position and Orientation", IEEE SENSORS JOURNAL, IEEE SERVICE CENTER, NEW YORK, NY, US, Bd. 9, Nr. 5, 1. Mai 2010 (2010-05-01), Seiten 903-913, XP011306917, ISSN: 1530-437X

# EP 3 010 413 B1

**Beschreibung**

Die Erfindung betrifft eine Vorrichtung mit einem Sensorarrangement, das magnetische oder magnetisierte orale Darreichungsformen nach der oralen Einnahme detektieren kann, des Weiteren deren Auflösung über die Abnahme oder das Verschwinden des magnetischen Feldes der oralen Darreichungsform verfolgt, und mit einer Logbuchfunktion, die eine subjektive Bewertung vonseiten des menschlichen Trägers des Sensorarrangements bei oder nach der oralen Einnahme festhält.

Im Stand der Technik ist die Erforschung der Ursache einer Krankheit, Allergie oder mangelhafter Befindlichkeit des Menschen an eine stationäre Untersuchung gebunden, an den Einsatz eines stationären Instrumentariums, an einen engen Zeitraum, währenddessen das Instrumentarium eingesetzt ist und sich der Mensch unter ärztlicher Beobachtung befindet. Anstelle eines Arztes kann auch jede andere spezialisierte Person gesehen werden, die das Wohlbefinden des Menschen bewahrt oder wieder herstellt, zum Beispiel ein Pharmazeut oder Therapeut jeglicher Fachrichtung.

Die Patentschrift US 7,698,156 B2 offenbart eine Vorrichtung zur Erfassung medizinischer Daten und ein Verfahren, die Datenströme eindeutig zu identifizieren. Sie erlaubt es, die von einzelnen medizinischen Geräten erzeugten Datenströme zu unterscheiden, festzuhalten, drahtlos zu übertragen und mit ihnen eine Grundlage für eine Diagnose und/oder Medikation des Patienten bereit zu stellen. Allerdings ist die Vorrichtung stationär, sie misst nur die körperlichen Funktionen des Patienten, und erfasst diese bedarfsweise zusammen mit der Zeit, z.B. dem Datum. Darüber hinaus erfassen konventionelle technische Hilfsmittel nichts weiter, was für den Fachmann auch nicht anders zu erwarten ist. Besonders in dem Zeitraum, bevor dieser Stand der Technik eingesetzt wird, fehlen belastbare Aussagen darüber, welche Zusammenhänge zwischen der Befindlichkeit des Menschen und messbaren Größen oder zeitlich erfassbaren Begebenheiten bestehen.

Die Patentanmeldung US 2010/0322859 A1 offenbart eine Vorrichtung mit einem Detektorsystem, die das Ereignis der Einnahme eines einzunehmenden Medikamentes erfasst. Dazu muss das Magnetfeld eines Permanentmagneten, der an dem Medikament angebracht ist, eine gewisse Stärke überschreiten. Das Ereignis wird festgestellt, indem eine Mikroelektronik mit Hilfe eines Algorithmus das Magnetfeld des Permanentmagneten mit Schwellwerten vergleicht.

Mikroelektronik und Algorithmus müssen an den Eigenschaften des Permanentmagneten sowie an den Einfluß des störenden Erdmagnetfeldes und weiterhin an den Bewegungen des menschlichen Trägers kalibriert sein. Daher müssen mehrere Sensoren mit eingespeicherten Schwellwerten angebracht sein, nämlich mehrere Sensoren mit horizontalen und vertikalen Schwellwerten. Diese Sensoren müssen außerdem gleichmässig verteilt in einem Halsband angebracht sein. Somit wird eine Vorrichtung vorgeschlagen, die den Zeitpunkt der Passage des Permanentmagneten erfasst.

Eine Herausforderung kann darin gesehen werden, dass sich der Mensch stets zunächst einer Gefährdung seiner Gesundheit oder Beeinträchtigung seines Wohlbefindens bewusst werden muss, bevor er einen Anlass wahrnimmt, sich einer Untersuchung und Messung seiner körperlichen Funktionen zu unterziehen und eine Diagnose zu erbitten. Diese Herausforderung kann mit der Frage verfeinert werden, inwieweit Gesundheit und/oder Wohlbefinden mit seiner Lebensweise zusammenhängen, insbesondere mit der Ernährung sowie mit der Einnahme von Nahrungsergänzungsmitteln, Genussmitteln, Drogen, aber auch homöopathischen Substanzen und/oder Medikamenten.

Aufgabe der Erfindung war es daher, eine Vorrichtung bereit zu stellen, die das Erfassen solcher Zusammenhänge ermöglicht, sowie ein Verfahren, mit dessen Hilfe eine Bewertung solcher Zusammenhänge gelingt.

Diese Aufgabe wurde überraschend mittels einer Vorrichtung gelöst, die das in der Patentanmeldung DE 10 2011 089 334.2 vorgestellte Detektorsystem und zusätzlich ein Logbuch umfasst, das zur Erfassung einer quantifizierten Bewertung vor, während und/oder nach der Erfassung des oder der magnetischen Körper vorgesehen ist.

Gegenstand der Erfindung ist also eine Vorrichtung, umfassend ein Detektorsystem zur Erfassung magnetischer Körper im menschlichen Organismus, wobei der magnetische Körper mit je einer Substanz fest verbunden ist, die vom menschlichen Organismus aufgenommen wird, wobei die Substanz ein Lebensmittel, Nahrungsergänzung oder Genußmittel ist,

wobei das Detektorsystem zumindest zwei Sensoranordnungen mit einem Gerät zur Aufzeichnung der von jeder Sensoranordnung gemessenen magnetischen Flussdichte aufweist, wobei jede Sensoranordnung von der oder den übrigen Sensoranordnungen einen Abstand von 0,5 bis 50 cm aufweist, und zumindest zwei Sensoranordnungen in einem Winkel von 0 bis 45 ° gegeneinander verkippt sind, und

ein Logbuch zur Erfassung einer quantifizierten Bewertung vor, während und/oder nach der Erfassung des oder der magnetischen Körper aufweist,

wobei die Sensoranordnungen in zumindest einem Gurt, der Kleidung, und/oder Schmuckgegenstand oder - gegenständen, in einem Armband, in einer Armbanduhr integriert, oder mittels Saugnapf oder Befestigungshilfe direkt am Körper fixiert sind, und das Logbuch am Körper mitgeführt ist,

und das Detektorsystem dadurch gekennzeichnet ist, dass

jede Sensoranordnung einen, zwei, oder drei anisotrope Magnetwiderstandssensoren aufweist, deren Achsen der leichten Magnetisierung in paarweise unterschiedliche Richtungen weisen, und jede Sensoranordnung einen Vektor liefert, der sich aus den Meßsignalen der AMR-Sensoren zusammensetzt, wobei die Vorrichtung dazu eingerichtet ist, die folgenden Schritte durchzuführen:

(a) zumindest einmaliges Aufschalten eines Set- und Reset-Impulses auf jeden anisotropen Magnetwiderstandssensor,
(b) Verstärkung der Meßsignale jedes AMR-Sensors über eine geeignete Signalaufbereitung und über zumindest einen Tiefpassfilter,
(c) Bestimmung und Aufzeichnung der Differenz der Beträge der Vektoren der magnetischen Flussdichten jeder Sensoranordnung, und/oder Bestimmung und Aufzeichnung des Winkels Φ zwischen den Vektoren aus den Meßsignalen der AMR-Sensoren, und
(d) zeitgleich mit einem der Schritte (a), (b) oder (c), oder nach Ablauf einer Zeit (T) im Anschluss an Schritt (c) zumindest einmalige Erfassung der quantifizierten Bewertung im Logbuch, die vom menschlichen Träger der Sensoranordnungenen vorgenommen wird.

Diese Vorrichtung hat den Vorteil, bewusste Aspekte ihres menschlichen Trägers, nämlich die quantifizierte Bewertung, und zumindest eine objektive Größe, nämlich die von dem magnetischen Körper gemessene magnetische Flussdichte erfassbar zu machen.

Die Erfindung wird im Folgenden näher erläutert.

Das Logbuch der erfindungsgemäßen Vorrichtung ist für Einträge vorgesehen, die der Träger der erfindungsgemäßen Vorrichtung aus eigenem Willen manuell im Sinne eines digitalen, elektronischen Tagebuches oder Notizbuches vornimmt. Das Logbuch kann ein mobiler Computer sein, vorzugsweise ein handelsübliches mobiles Telefon, PDA, Kleincomputer, Datalogger mit Sender, und/oder eine Eingabeeinheit, wobei das Logbuch elektronisch mit der Sensoranordnung verbunden ist. Somit kann der menschliche Träger der Vorrichtung das Logbuch handhaben, wie dies den Gewohnheiten des Trägers eines mobilen Computers entspricht.

Das Logbuch kann über eine Bluetooth Schnittstelle verfügen, um vom Sensorarrangement aufgezeichnete Daten zusammen mit den Logbuch Einträgen abrufen zu können.
Die Sensoranordnungen der erfindungsgemäßen Vorrichtung sind in zumindest einem Gurt, der Kleidung, und/oder Schmuckgegenstand oder - gegenständen, in einem Armband, zum Beispiel in einer Armbanduhr integriert, oder sind mittels Saugnapf oder Befestigungshilfe direkt am Körper fixiert, und das Logbuch ist am Körper mitgeführt.

Der Vorteil einer solchen Vorrichtung ist die Mobilität, denn der menschliche Träger kann die erfindungsgemäße Vorrichtung bei allen täglichen Aktivitäten ohne Einschränkung seiner Beweglichkeit mit sich führen.

Besonders bevorzugt kann der Gurt, in welchem die Sensoranordnungen integriert sein können, ohne Hilfe eines Dritten am Menschen angelegt werden. Dieser Gurt kann beispielsweise ein Gürtel sein, der seinen Träger nur minimal in seinen alltäglichen Bewegungen einschränkt. Der Gurt kann vorteilhaft ein kombinierter Brust- und Schultergurt sein. Besonders vorteilhaft kann der kombinierte Brust- und Schultergurt ein Gurtsystem sein, das aus dem Klettersport bekannt ist. Der kombinierte Brust- und Schultergurt hat den Vorteil, die Sensoranordnungen mit hoher Genauigkeit relativ zur Speiseröhre und dem Magen-Darm-Trakt zu positionieren. Das Gurtsystem hat zusätzlich den besonderen Vorteil, die Sensoranordnungen der erfindungsgemäßen Vorrichtung besonders genau jeweils in definiertem Abstand und deren Achsen der leichten Magnetisierung in definiertem Winkel zu halten. Der Gurt erlaubt seinem Träger die volle Beweglichkeit bei den alltäglichen Verrichtungen, insbesondere bei Betätigungen in Beruf und Freizeit. Die erfindungsgemäße Vorrichtung kann auch an jedem Gegenstand mitgeführt sein, der in der Nähe des Körpers ist oder am Körper des Menschen mitgeführt wird, zum Beispiel an einem Rollstuhl, Rollator, einer Wiege, Liege, oder an Krücken angebracht, oder in einer Armbanduhr, in einem Armband, Kette oder Schmuckgegenstand integriert.
Weist eine Sensoranordnung der erfindungsgemäßen Vorrichtung lediglich einen AMR-Sensor auf, wird diese im Rahmen der Erfindung auch "einkanalig" genannt, bei drei AMR-Sensoren entsprechend "dreikanalig". Weist eine Sensoranordnung beispielsweise drei AMR-Sensoren auf, deren leicht magnetisierbare Achsen wie die Koordinatenachsen x, y, und z eines kartesischen Koordinatensystems angeordnet sind, so sind die Komponenten des Vektors dieser Sensoranordnung die Meßsignale in x, y, bzw. in z-Richtung die Signale $S_x$, $S_y$, und $S_z$. Sie sind das Maß für die magnetische Flussdichte in Richtung der Koordinatenachsen.
Die Achsen der leichten Magnetisierung einer Sensoranordnung treffen sich in einem gedachten Punkt, dem Ursprung der jeweiligen Sensoranordnung. Der Abstand zwischen diesen Ursprüngen, bzw. bei drei Sensoranordnungen der paarweise Abstand zwischen diesen Ursprüngen, ist im Rahmen der Erfindung der Abstand bzw. der paarweise Abstand zwischen den Sensoranordnungen.

Die Achsen der leichten Magnetisierung der zweiten Sensoranordnung liegen jeweils parallel zu den Koordinatenachsen x, y, und z, oder in einem Winkel dazu. Im Rahmen der Erfindung ist dieser Winkel wie folgt definiert: Die Achsen der leichten Magnetisierung jeder Sensoranordnung liegen auf je einem gedachten Kegelmantel eines Raumwinkels. Im Rahmen der Erfindung ist der Winkel, in dem die zwei Sensoranordnungen des erfindungsgemäßen Detektorsystems gegeneinander verkippt sind, der Winkel zwischen den Mittenachsen der Kegel der Sensoranordnungen.

Wird das Detektorsystem in einem Gurt, Armband, oder Gegenstand in der Nähe des Körpers mitgeführt, liegt der Winkel im Rahmen der Genauigkeit, mit der der Gurt eingestellt werden kann, in der Ebene, die durch die Ursprünge der Sensoranordnungen und den Eintrittspunkt der Speiseröhre in den Magen definiert ist. Besonders hohe Genauigkeiten werden erreicht, wenn dieser Gegenstand ein aus dem Klettersport bekanntes Gurtsystem ist.

Weist die erfindungsgemäße Vorrichtung zwei Sensoranordnungen auf, werden die Richtungen und die Signale durchnummeriert. Demnach erhält man in den Richtungen x1, y1, z1 bzw. x2, y2, z2 die Signale $S_{x1}$, $S_{y1}$, und $S_{z1}$ bzw. $S_{x2}$, $S_{y2}$, und $S_{z2}$, aus denen man die Vektoren $\boldsymbol{S_1}$ und $\boldsymbol{S_2}$ bildet:

$$\boldsymbol{S_1} = (S_{x1}, S_{y1}, S_{z1}),$$

und

$$\boldsymbol{S_2} = (S_{x2}, S_{y2}, S_{z2}).$$

Weist beispielsweise die erste der Sensoranordnungen der erfindungsgemäßen Vorrichtung nur einen AMR-Sensor auf, nämlich in Richtung x1, vereinfacht sich der Vektor $\boldsymbol{S_1}$ zu $\boldsymbol{S_1} = (S_{x1}, 0, 0)$.

Die erfindungsgemäße Vorrichtung hat den Vorteil, diese Vektorkomponenten jeweils so genau zu messen und derart auswertbar zu machen, dass bei der Bewegung der Sensoranordnung durch den Träger die Schwankung des Betrages dieser Vektoren entweder klein bleibt, oder soweit bekannt ist, dass die durch einen magnetischen Körper verursachte Veränderung der Messwerte erfasst wird. Somit wird der Einfluss äußerer Störquellen erkannt und beseitigt, oder kann aus dem Meßsignal gefiltert werden.

Die Beträge der Vektoren, abgekürzt mit $|\boldsymbol{S_1}|$ und $|\boldsymbol{S_2}|$, errechnet man auf bekannte Weise:

$$|\boldsymbol{S_1}| = (S_{x1}^2 + S_{y1}^2 + S_{z1}^2)^{1/2},$$

$$|\boldsymbol{S_2}| = (S_{x2}^2 + S_{y2}^2 + S_{z2}^2)^{1/2}.$$

Bei geringem Abstand zwischen den Sensoranordnungen ergeben sich in homogenen Feldern gleiche Messwerte. Ein magnetischer Körper mit geringer magnetischer Induktion in der Nähe der Sensoren beeinflusst durch sein mit dem Abstand zum Sensor rasch abklingendes Magnetfeld bei unterschiedlichen Abständen zu den Sensoren deren Messwerte unterschiedlich. Da aber jede Sensoranordnung einen Vektor liefert, der sich aus den Meßsignalen der AMR-Sensoren zusammensetzt, hat die erfindungsgemäße Vorrichtung den Vorteil, dass sich die Nähe des magnetischen Körpers zu den Sensoranordnungen auf den Winkel zwischen den gemessenen Vektoren auswirkt. Bewegt sich der magnetische Körper, ändert sich dieser Winkel.

Die Messempfindlichkeit kann durch vorteilhafte Ausführungen der erfindungsgemäßen Vorrichtung erhöht werden.

Vorzugsweise weist zumindest einer, bevorzugt jeder AMR-Sensor der Vorrichtung 4 Barberpole-Elemente auf, die zu einer Wheatstonebrücke oder einer Wheatstonebrückenersatzschaltung zusammengeschaltet sind. Die Achse der leichten Magnetisierung ist dann die Resultierende der Achsen der leichten Magnetisierung der einzelnen Barberpole-Elemente. Äußere Magnetfelder verstimmen eine solche Wheatstonebrücke viel stärker, als z.B. eine Widerstandsbrücke mit nur einem Barberpole-Element und drei konventionellen ohmschen Widerständen. Die Empfindlichkeit einer Wheatstone-Brücke aus 4 Barberpole-Elementen ist demnach erhöht.

In der Fachwelt ist bekannt, dass die Kennlinie des AMR-Sensors durch starke Magnetfelder verändert werden kann, weil sich Domänen des anisotropen Materials umbilden oder verformen, oder weil sich deren Wände in dem Material verlagern. Diesem Effekt wird durch zumindest einen Set- oder/und Reset-Impuls entgegen gewirkt, der einmalig vor der Messung, vorzugsweise mehrmals während der Messung, besonders bevorzugt periodisch während der Messung über einen Set-Reset-Strap aufgegeben wird. Die Wirkung periodisch aufgegebener Set- und/oder Reset-Impulse besteht in der Sicherstellung der optimalen Kennlinie der AMR-Sensoren.

Ein Alternieren des Set- und Reset-Impulses, im Rahmen der Erfindung *"Flippen"* genannt, ermöglicht eine Beseitigung von Offsetfehlern durch Differenzbildung der nach jedem Impuls gemessenen Signale. Des Weiteren werden thermische, elektrische, und/oder solche Einflüsse eliminiert, die zum Beispiel bei der Erwärmung des AMR-Sensors auftreten.

Ebenfalls wird mit dem *Flippen* eine automatische Einstellung des Arbeitspunktes des nachfolgenden Verstärkers ermöglicht, die im Rahmen der Erfindung *"switching feedback"* genannt wird. Neben dem Tastverhältnis ist auch das sichere Erreichen der Sättigungsinduktion durch die Set- und Reset-Impulse wichtig.

Bei der Differenzbildung muss der Arbeitspunkt für den nachfolgenden Verstärker eingestellt werden. Ungenauigkeiten dieser Einstellung wirken sich bei sehr großer Aussteuerung durch eine unsymmetrisch einsetzende Begrenzung des Signals aus.

Außerdem kann das erfindungsgemäße Detektorsystem einen Offset-Strap aufweisen. Der Strom durch den Offset-Strap kann durch eine Treiberschaltung geliefert werden, die z. B. einen Verstärker in Brückenschaltung als wesentliches Element enthalten kann. Der Offset-Strap ermöglicht die Kompensation der zu messenden Feldkomponente durch Erzeugung eines Feldes mit entgegengesetzter Ausrichtung. Ohne einen Offset-Strap muss bei der Messung der magnetischen Flussdichte die Nichtlinearität der Sensorkennlinie und außerdem die Querempfindlichkeit der AMR-Sensoren berücksichtigt werden. Die Querempfindlichkeit besteht in der Wirkung hoher Werte der magnetischen Flussdichte in sowohl einer Achsenrichtung, als auch auf den Messwert eines orthogonal dazu ausgerichteten AMR-Sensors.

Mit Offset-Strap jedoch wird die Brückenspannung des Sensors in einer Regelschleife durch Einspeisung eines Stroms in den Offset-Strap minimiert. Der für den Brückenabgleich erforderliche Strom im Offset-Strap ist ein Maß für das zu messende Feld. Dadurch wird stets in demjenigen Arbeitspunkt der Sensorkennlinie gemessen, in dem die Empfindlichkeit und Linearität ihr Maximum haben und gleichzeitig die Querempfindlichkeit verschwindet. Das erfindungsgemäße Detektorsystem ist deshalb für jede alltägliche Umgebung geeignet. Der Offset-Strap ist mit dem "Offset Strap Treiber" beschaltet. Allgemein können Nichtlinearitäten und Querempfindlichkeiten bei der Kalibrierung erfasst und das Messergebnis entsprechend korrigiert werden. Dadurch ist auch ein Betrieb ohne Ansteuerung des Offset-Straps bei minimiertem Energieverbrauch möglich.

Es gibt eine weitere Alternative zu der Kompensation der zu messenden Feldkomponente durch Erzeugung eines Feldes mit entgegengesetzter Ausrichtung mittels der Einspeisung eines Stroms in den Offset-Strap. Dabei kann zumindest ein, vorzugsweise jeder AMR-Sensor der erfindungsgemäßen Vorrichtung mit einer alternativen Schaltung ausgestattet sein.

Bei dieser Ausführungsform der Vorrichtung wird die Brückenspannung des Sensors nicht in einer Gegenkopplungsschaltung auf den Sollwert Null ausgeregelt. Stattdessen wird mittels eines DA-Wandlers und eines Verstärkers ein definierter Strom in den Offset-Strap in der Art eingespeist, dass ein bestimmter Aussteuerbereich der Sensorbrücke nicht verlassen wird.

Bei einer weiteren Möglichkeit der Ausführung der erfindungsgemäßen Vorrichtung kann der Aussteuerbereich der Sensorkennlinie in eine Anzahl von Segmenten unterteilt werden, beispielsweise bei einem DA-Wandler mit 8 Bit Auflösung in 256 Segmente. Zur Gewährleistung einer kontinuierlichen Messung bei sich ändernder magnetischer Feldstärke können die Segmente so gewählt werden, dass eine ausreichende Überlappung benachbarter Segmente vorliegt. Jedes dieser Segmente kann dann mit nur einem kleinen Aussteuerbereich um den optimalen Arbeitspunkt des AMR-Sensors versehen werden. Die Verringerung des Aussteuerbereiches verringert die Querempfindlichkeit und die Auswirkungen von Nichtlinearitäten der Kennlinie. Auf die vollständige Korrektur von Nichtlinearität und Querempfindlichkeit wird verzichtet. Dafür jedoch wird eine verbesserte Amplitudenauflösung der Messung durch die Kombination aus AD-Wandler und Segmentierung der Kennlinie erhalten.

Dafür müssen für jedes der Segmente eines AMR-Sensor Messbereiches die Parameter der Approximation durch jeweils eine Gerade mit ihrer dazu gehörenden Steigung und Höhenabschnitt bestimmt werden. Die Steigungen und Höhenabschnitte der Segmente werden über die Kalibrierungsdateien der Sensoren bereitgestellt. Wird das erfindungsgemäße Detektorsystem nun im täglichen Gebrauch bewegt, etwa durch die alltäglichen Bewegungen seines Trägers, so wird der definierte Strom und damit die Approximation ständig nachgeführt.

Entsprechend der Geschwindigkeit, mit der die Bewegungen erfolgen, ist eine hohe Abtastrate vorteilhaft, so dass ohne Übersteuerungen eine kontinuierliche Messung realisiert ist.

Der Vorteil dieser Variante besteht darin, dass bei entsprechend schneller Abtastung die Offsetstraps nur mit einem sehr kleinen Tastverhältnis betrieben werden müssen. Dadurch werden der Leistungsbedarf und die Eigenerwärmung der Sensoren und damit verbundene Offsetprobleme stark verringert.

Außerdem kann durch die Verwendung schneller AD- und DA-Wandler bei der für die kontinuierliche Messung im Magnetfeld erforderlichen Messfrequenz die für die einzelne Messung benötigte Zeit gering gehalten werden. Damit ist es möglich, die Offsetstraps nur während der für die Messwerterfassung erforderlichen Zeit anzusteuern. Erfolgt die Ansteuerung der Offsetstraps zum Beispiel nur mit einem Tastverhältnis von 0,1, zum Beispiel bei 1 ms Messdauer und einem zeitlichen Abstand zwischen aufeinanderfolgenden Messungen von 10 ms, so verringert sich die Verlustleistung.

Dadurch wird weniger Wärme entwickelt und somit die Drift der Meßsignale vermindert oder ganz unterdrückt.

Für die Brauchbarkeit der erfindungsgemäßen Vorrichtung mit zwei Sensoranordnungen ist zu beachten, dass die Speiseröhre eine Länge von 20 bis 30 cm hat und von einem geschluckten Objekt in 5 bis 10 s durchlaufen wird. Daraus ergibt sich ein Geschwindigkeitsbereich bei der Speiseröhrenpassage von 2 bis 6 cm/s und damit ein entsprechend schnell veränderliches Signal für das Detektorsystem. Der Frequenzbereich des Nutzsignals deckt sich also mit dem Frequenzbereich, den einige der externen Störsignale besitzen. Im Rahmen der Erfindung werden *"externe Störsignale"* solche Signale genannt, die von magnetischen Flüssen herrühren, die den Träger umgeben und in denen er sich - zwangsweise - bewegt, zum Beispiel im Erdmagnetfeld oder in der Umgebung magnetischer Objekte wie z. B. Fahrzeuge. Aufgrund externer Störsignale würde man eine Unterscheidbarkeit zwischen einer Passage eines magnetischen Objektes durch die Speiseröhre und magnetischen Flüssen anderer Objekte nicht erwarten. Insbesondere führt eine Stand der Technik gemäße Filterung des Meßsignals nicht zum Erfolg.

Eine bekannte Möglichkeit zur Ausschaltung externer Störungen bietet sich durch die Auswertung von Auto- und Kreuzkorrelationsfunktionen von Sensoren, die in einem festen Abstand zu einander positioniert sind. Die Kreuzkorrelation beschreibt die Korrelation zweier Signale in Abhängigkeit von der Zeitverschiebung zwischen diesen Signalen. Bei der Autokorrelation wird die Korrelation eines Signals mit sich selbst berechnet. Die Autokorrelationsfunktion hat stets ein Maximum bei der Verschiebung 0. Wird ein Signal mit einer Verzögerung durch zwei sonst gleiche Sensoren aufgenommen, so ist das Maximum der Kreuzkorrelationsfunktion bei sonst gleicher Form um die Verzögerung gegenüber dem Maximum der Autokorrelationsfunktionen verschoben.

Eine wesentliche Voraussetzung für die Identifizierung einer Kapselpassage durch die Speiseröhre ist es, dass die Sensoranordnungen eine zeitversetzte Komponente der Signale erfassen können. Das Problem, welches jedoch verbleibt, ist von der Bewegung der Sensoranordnung im umgebenden Erdmagnetfeld verursacht, die die Brauchbarkeit der erfindungsgemäßen Vorrichtung ja gerade ausmacht.

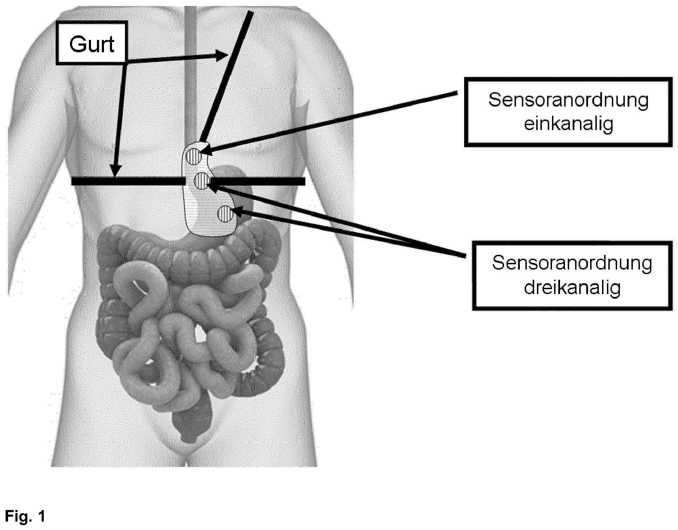

Trotz der Vielfalt magnetischer Flüsse zahlreicher Objekte, beispielsweise von Fahrzeugen, metallischen Möbeln, stromführenden Leitungen und Ähnlichem mehr, erkennt die erfindungsgemäße Vorrichtung eindeutig solche Flüsse, die von dem magnetischen Körper im menschlichen Organismus stammen, wenn der Abstand zwischen zwei Sensoranordnungen von 2 bis 6 cm gewählt ist. Durch ungleiche Orte der Sensoranordnungen werden externe Magnetfelder erkannt, die nicht von dem magnetischen Körper im menschlichen Organismus stammen. Bevorzugt sind die Sensoranordnungen über Speiseröhre oder Brustbein und Magen senkrecht oder waagerecht fixiert. Die *Fig. 1*. zeigt die erfindungsgemäße Vorrichtung mit drei Sensoranordnungen in einem kombinierten Brust- und Schultergurt, getragen am Menschen. Das Logbuch und das oder die Geräte zur Aufzeichnung der von jeder Sensoranordnung gemessenen magnetischen Flussdichte sind nicht abgebildet. In dieser beispielhaften Ausführungsform ist die Sensoranordnung in Nähe der Speiseröhre einkanalig, die beiden anderen Sensoranordnungen sind dagegen dreikanalig ausgeführt. Die lediglich einkanalige Ausführung der Sensoranordnung in Nähe der Speiseröhre vereinfacht den Aufbau und mindert den Energiebedarf der erfindungsgemäßen Vorrichtung. Außerdem nutzt diese einkanalige Ausführungsform die Möglichkeit, dass der magnetische Körper nicht kugelsymmetrisch ausgeführt sein muss, sondern zum Beispiel zylindersymmetrisch ausgeführt sein kann, und das vom ihm erzeugte Magnetfeld sich dadurch während des Durchlaufens der Speiseröhre relativ zur einkanaligen Sensoranordnung bewegt, ohne zu rotieren.

Es kann auch vorteilhaft sein, den Anteil der störenden Umgebungsfelder durch die Subtraktion eines gleitenden Mittelwerts zu entfernen und den Abstand zwischen den Sensoranordnungen mit 2 cm zu wählen. Mit den gefilterten Signalen können dann deren Auto- und Kreuzkorrelationsfunktionen berechnet werden. Anhand der Unterschiede der Amplituden und der Lage der Maxima kann dann die Passage eines magnetischen Körpers erkannt werden.

Falls die erfindungsgemäße Vorrichtung zwei oder drei Sensoranordnungen aufweist, kann diese für eine Erfassung des magnetischen Körpers im Magen verwendet werden.

Die langsame Desintegration des magnetischen Körpers führt zur Abschwächung von dessen magnetischer Flussdichte. Bewegungen des Trägers und Lageveränderungen des magnetischen Körpers, zum Beispiel durch die Peristaltik, führen zu Schwankungen des Messwertes. Obwohl im Allgemeinen keine Aussagen über das superponierte Bewegungsmuster aus Peristaltik und magnetischem Körper möglich sind, führt die Vorrichtung mit drei Sensoranordnungen zum Erfolg. Vorteilhaft ist des Weiteren, die Vorrichtung mit einer Tiefpassfilterung als Maßnahme für die Signalverarbeitung auszustatten.

Der magnetische Körper kann so ausgeprägt sein, dass er über die orale Einnahme verabreicht, insbesondere durch den Menschen geschluckt werden kann. Die Gestalt dieses magnetischen Körpers wird im Rahmen der Erfindung auch *"orale Darreichungsform"* genannt. Diese kann eine Kapsel oder eine Kapsel mit Funktion sein, wobei die Funktion ausgewählt ist aus Diagnostikum und/oder Arzneiform. Die Kapsel kann weiterhin bevorzugt eine Tablette sein, die die Speiseröhre vorzugsweise in Längsrichtung passiert. Die Darreichungsform weist zumindest einen magnetischen, vorzugsweise einen para-, superpara-, ferri-, und/oder ferromagnetischen Anteil auf, vorzugsweise zumindest einen Magnetit enthaltenden Kern und/oder Mantel. Der magnetische Anteil kann magnetisch orientierbare oder magnetisierbare Partikel aufweisen, vorzugsweise Magnetit ($Fe_3O_4$) oder Maghemit ($Fe_2O_3$). Magnetit und Maghemit gelten als toxikologisch und pharmakologisch unbedenklich und werden unter anderem als nicht toxische, unlösliche Pigmente in Lebensmittel oder Arzneiformen eingesetzt.

Geeignet können gegebenenfalls auch andere magnetisch orientierbare Partikel wie die Ferrite $MnFe_2O_4$ oder $MgFe_2O_4$ sein. Der magnetische Anteil des magnetischen Körpers kann in dem Bereich von 0,05 bis 80 mg, bevorzugt von 2 bis 70, bevorzugt 4 bis 60, insbesondere 6 bis 50 mg an magnetisch orientierbaren oder magnetisierbaren Partikeln betragen. Die mittleren Teilchengrößen der magnetisch orientierbare Partikel können z. B. im Bereich von 1 nm bis 1 mm, bevorzugt von 100 nm bis 100 $\mu$m liegen.

Die orale Darreichungsform kann ebenfalls bevorzugt eine Kapsel sein, eine Tablette, ein Stäbchen, eine überzogene Tablette, ein Schmelzextrudat, oder ein Körper mit einem eingearbeiteten magnetischen Film sein.

Damit erfasst die erfindungsgemäße Vorrichtung, bei welcher eine Sensoranordnung orthogonal zur Hauptachse der Darreichungsform ausgerichtet ist, eine markante Änderung des Messwertes bei deren Passage.

Die zeitliche und die räumliche Skala, auf der die Meßsignale zumindest der beiden Sensoranordnungen liegen, sind durch die Geschwindigkeit gegeben, mit der die orale Darreichungsform das erfindungsgemäße Detektorsystem passiert, und durch die Abstände bzw. paarweisen Abstände der Sensoranordnungen. Obwohl sich, wie oben bereits gesagt, eine Vielzahl magnetischer Flussdichten überlagern und die eigentlich interessierende Flussdichte sehr klein und zeitlich und räumlich inhomogen ist, wurde erkannt, dass diese sich mit dem erfindungsgemäßen Detektorsystem sicher erfassen lässt.

Da die erfindungsgemäße Vorrichtung ein Logbuch aufweist, lassen sich zusammen mit den interessierenden Flussdichten ebenfalls subjektive Einträge zeitnah und/oder zeitgleich mit dem Passieren der oralen Darreichungsform erfassen.

Die erfindungsgemäße Vorrichtung ist dazu eingerichtet, die folgenden Schritte durchzuführen:

(a) zumindest einmaliges Aufschalten eines Set- und Reset-Impulses auf jeden anisotropen Magnetwiderstandssensor,

(b) Verstärkung der Signale jedes AMR-Sensors über eine geeignete Signalaufbereitung und über zumindest einen Tiefpassfilter,

(c) Bestimmung und Aufzeichnung der Differenz der Beträge der Vektoren der magnetischen Flussdichten jeder Sensoranordnung, und/oder Bestimmung und Aufzeichnung des Winkels $\Phi$ zwischen den Vektoren aus den Meßsignalen der AMR-Sensoren,

und

(d) zeitgleich mit einem der Schritte (a), (b) oder (c), oder nach Ablauf einer Zeit (T) im Anschluss an Schritt (c) zumindest einmalige Erfassung der quantifizierten Bewertung im Logbuch, die von dem menschlichen Träger der Sensoranordnungen vorgenommen wird.

Bei der Erfassung von Messwerten werden dynamische Störeinflüsse gemindert, indem die Verfälschung von Offsetwerten, z. B. durch vorbeifahrende Fahrzeuge, oder das Ein- und Ausschwingverhalten von eingesetzten Filtern verringert wird. Die simultane und/oder zeitnahe Erfassung subjektiver Kriterien ermöglicht Korrelationen, die nicht allein anhand objektiver Messdaten zugänglich sind.

Im Schritt (a) sind die Set- und Reset-Impulse alternierend, gleichbedeutend damit, dass diese zyklisch aufgepulst werden. Sie sollten mit einer Stromimpulsstärke aufgegeben werden, bei der die Sättigungsmagnetisierung jeweils erreicht und damit die Steigung der Kennlinie kontrolliert wird. Die Stromimpulsstärke schwankt je nach Bauelement auf eine dem Fachmann bekannte Weise.

Im Schritt (b) können bevorzugt Gaußfilter, Besselfilter zum Unterdrücken von Überschwingern oder Welligkeiten im Signal eingesetzt werden. Um rasche und langsame Änderungen in den Signalen zu trennen, sind dem Fachmann bekannte Bandpassfilter eine bevorzugte Art der Signalaufbereitung. Periodische elektromagnetische Störungen mit Frequenzen von 16,7 Hz, z.B. beim elektrifizierten Bahnbetrieb, bzw. 50 Hz, der Netzfrequenz, können durch die Wahl der Abtastrate und der Integrationszeit von 60 ms oder vielfachen bei der Datenerfassung unterdrückt werden. Die Integrationszeit ist bei abweichenden Frequenzen der periodischen Störungen entsprechend anzupassen.

Um elektromagnetische Störeinstrahlungen der Frequenzbereiche von 16 bis 50 Hz auszufiltern, sind 2 Anordnungen bevorzugt, bei denen die Integrationskonstante mindestens 60 msec beträgt. Vorzugsweise werden so die Abtastfrequenzen an verschiedene, periodisch auftretende Störquellen angepasst.

Das Maß der magnetischen Flussdichte in x-, y-, und z-Richtung beim Schritt (c) sind die aus der Verstimmung der Wheatstone Brücken der AMR-Sensoren in der jeweiligen Richtung abfallenden Spannungen. Der Fachmann nimmt an, dass sich in der Differenz $\Delta_0$ der Vektoren zweier Sensoranordnungen,

$$\Delta_0 = S_1 - S_2,$$

die Anteile homogener magnetischer Flussdichten gerade aufheben. Der Einfluss störender externer Felder, räumlich kaum veränderlich, wäre damit kompensiert, und es bliebe im Wesentlichen das Feld des magnetischen Körpers im Träger allein übrig. Die beiden Sensoranordnungen dürfen dabei jedoch nicht oder nur wenig gegeneinander verkippt sein, gleichbedeutend mit dem Winkel 0 °. Magnetische Flussdichten raum- und zeitversetzter Ereignisse werden jedoch überraschend auch bei größeren Winkeln erfasst, wenn anstelle $\Delta_0$ der skalare Wert $\Delta$ gebildet wird:

$$\Delta = |S_1| - |S_2|$$

Dies vereinfacht die Montage der Sensoranordnungen in dem Gurt der erfindungsgemäßen Vorrichtung und erspart außerdem umständliche Anpassungen der Lage der Sensoranordnungen an unterschiedliche Proportionen des Trägers. In einem Diagramm des Wertes $\Delta$ als Funktion der Zeit werden so charakteristische Linienformen erkannt, die zum Beispiel dem Schlucken des magnetischen Körpers, seinem Gang durch die Speiseröhre, somit die Passage der Sensoranordnungen, und seiner Bewegungen aufgrund der Peristaltik bei der Verdauung zugeordnet werden.

Um diese Zuordnung vornehmen zu können, ist die Filterung des Meßsignals nicht ausreichend. Zwar kennt der Stand der Technik eine Möglichkeit zur Ausschaltung externer Störungen durch die Auswertung von Auto- und Kreuzkorrelationsfunktionen von Sensoren, die in einem festen Abstand zueinander positioniert sind. Wird ein Signal mit einer Verzögerung durch zwei sonst gleiche Sensoren aufgenommen, so ist das Maximum der Kreuzkorrelationsfunktion bei sonst gleicher Form um die Verzögerung gegenüber dem Maximum der Autokorrelationsfunktionen verschoben. Damit nunmehr ein Zeitversatz zwischen Auto- und Kreuzkorrelation der Sensorsignale erkannt werden kann, darf der von der oralen Darreichungsform verursachte Signalanteil nicht durch externe Magnetfelder überdeckt werden. Dazu aber würde man die externen Störungen weitgehend beseitigen müssen. Hierzu wird beispielsweise die Differenzbildung zwischen aktuellem Signal und einem Mittelwert genutzt. Diesen Mittelwert muss man der aktuellen Situation anpassen und zum Beispiel als sogenannten "gleitenden Mittelwert (Moving Average)" gewinnen. Dies jedoch bedingt, dass der Proband während der Einnahme der oralen Darreichungsform weder schnelle rotatorische, noch rasche translatorische Bewegungen mit großer Amplitude vollzieht. Erst dann gewährleisten Sensoren gemäß Stand der Technik eine ausreichende Signaltrennung.

Natürlich kann man auch die Abtastrate erhöhen, so dass ohne Übersteuerungen eine kontinuierliche Messung realisiert wäre. Der Nachteil eines dann erhöhten Energiebedarfs kann wenigstens teilweise dadurch kompensiert werden, indem hohe Abtastraten nur bei interessanten, komplexen Ereignissen eingestellt werden, wie zum Beispiel beim Schlucken und/oder dem Zerfall des magnetischen Körpers. Solche interessanten, komplexen Ereignisse müssen von dem System allerdings erkannt werden. Dies aber bewerkstelligt die erfindungsgemäße Vorrichtung, nämlich anhand der Registrierung des genauen Zeitpunktes der Einnahme und, falls der menschliche Träger dies vorsieht, anhand zumindest eines Eintrages im Logbuch. Man kann sogar das im Stand der Technik bekannte Problem, dass schnelle rotatorische und/oder translatorische Bewegungen mit großer Amplitude, die nicht mit der oralen Darreichungsform zusammenhängen, in den Meßsignalen weiterhin sichtbar sind, mit Eintrag oder Einträgen ins Logbuch korrelieren. So liegen weiterführende Informationen über das Umfeld des Trägers und ebenfalls über Umstände Einnahme oraler Darreichungsformen, und/oder Informationen darüber vor, wie nicht mit der oralen Darreichungsform zusammenhängende Störungen zu kompensieren sind.

Das alternative Berechnen des von den Meßsignalvektoren eingeschlossenen Winkels $\Phi$ nach der Formel I,

$$\text{I} \qquad \Phi = \arccos(S_1 \cdot S_2 \,/\, |S_1||S_2|),$$

im Schritt (c) des Verfahrens ist eine Alternative, die oben genanntes Problem umgehen kann. Wir fanden, dass sich schnelle Bewegungen des Trägers und/oder rasche externe Flussänderungen der störenden Felder auf die relative Orientierung der Meßsignalvektoren zueinander weniger signifikant auswirken, als die Bewegung des magnetischen Körpers im Träger Organismus. Dies kann damit erklärt werden, dass die Quellen für externe Flussänderungen beide Meßsignalvektoren, oder bei drei Sensoranordnungen drei Meßsignalvektoren, in zumindest ungefähr gleiche Richtungen ablenken. Obwohl deren Beträge durchaus unterschiedlich verändert werden können, muss der Winkel zwischen je zwei Paaren der Meßsignalvektoren, auf die Zeit bezogen, in etwa der gleiche bleiben. Das ist gleichbedeutend damit, dass das umgebende Magnetfeld weiter entfernter Quellen seine Homogenität oder Inhomogenität in etwa beibehält. I erlaubt demnach das Ausblenden weiter entfernter Quellen magnetischer Flüsse unabhängig von deren zeitlichem Verhalten.

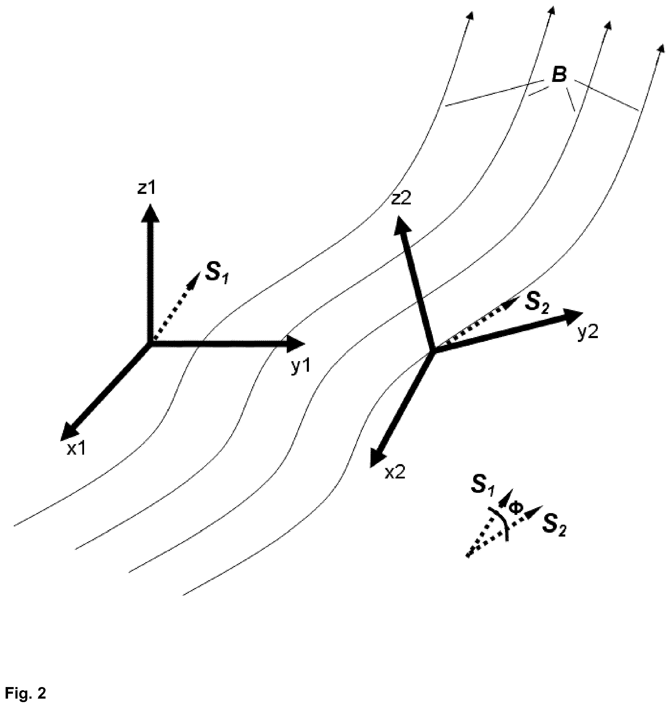

Die Situation bei der Durchführung des erfindungsgemäßen Verfahrens zeigt schematisch die *Fig. 2*. Die Hinweiszeichen bedeuten:

*B*        Feldlinien des störenden magnetischen Flusses
*S₁, S₂*   Vektoren $S_1 = (S_{x1}, S_{y1}, S_{z1})$ bzw. $S_2 = (S_{x2}, S_{y2}, S_{z2})$
$\Phi$        von den Meßsignalvektoren eingeschlossener Winkel nach der Formel **I**.

Unter der Annahme, dass Quellen des störenden magnetischen Flusses räumlich weiter entfernt sind, als der magnetische Körper bzw. die orale Darreichungsform, ist der Winkel zwischen den Vektoren $S_1$ und $S_2$ näherungsweise zeitlich konstant. Im besten Falle, nämlich in einem homogenen Magnetfeld, verschwindet dieser Winkel sogar konstant. Es wurde aber gefunden, dass störende Magnetfelder oft im Wesentlichen homogen sind. Ein Vorteil bei der Bestimmung des Winkels Φ ist die Unerheblichkeit fehlerhafter Ausrichtung einzelner oder aller AMR-Sensoren oder der Verkippung der Sensoranordnungen gegeneinander, wenn diese fehlerhafte Ausrichtung zeitlich konstant ist. Ein solcher Fehler macht sich in einem bedeutungslosen Offset im Φ/t - Diagramm bemerkbar, gleichbedeutend mit

$$\Phi = const$$

bezüglich der Zeit t.

In dem erfindungsgemäßen Verfahren kann im Schritt (b) zumindest ein Tiefpassfilter mit der Grenzfrequenz von 0,1 - 0,99 mHz, 1 mHz - 0,99 Hz, 1 Hz - 9,99 Hz, 10 Hz - 1 kHz, oder eine Kombination aus Tiefpassfiltern mit zumindest zwei verschiedenen Grenzfrequenzen eingesetzt werden. Dabei ist bevorzugt, die Filterung dem zu detektierenden Vorgang anzupassen, um Rauschen und/oder rasch veränderliche Störfelder, zum Beispiel durch Elektrogeräte, im Messsignal zu unterdrücken.

In dem erfindungsgemäßen Verfahren kann der Beitrag eines jeden AMR-Sensors oder das im Schritt (c) erhaltene Meßsignal durch einen Medianfilter gefiltert werden.

Des Weiteren kann in dem erfindungsgemäßen Verfahren die Aufzeichnung während der Durchführung des Schrittes (c) der erhaltenen Größe $\Delta$ und/oder Φ als Funktion der Zeit durch einen Datalogger oder ein anderes geeignetes, dem Fachmann bekanntes Gerät erfolgen, mit dem die erfindungsgemäße Vorrichtung ausgestattet ist. Diese Aufzeichnung kann kontinuierlich erfolgen, zum Beispiel während der Einnahme, der Passage und/oder der Zersetzung des magnetischen Körpers im Organismus des Trägers. Sie kann auch diskontinuierlich erfolgen, um beispielsweise Energie zu sparen.

Es wurde gefunden, dass viele alltägliche Quellen störender Felder charakteristische Linienformen in den $\Delta$/t - bzw. Φ/t - Diagrammen erzeugen. So können zum Beispiel vorbeifahrende Kraftfahrzeuge, elektrische Schaltvorgänge, durch Funken verursachte elektromagnetische Störungen, sowie stochastisch periodische Störungen und/oder Bürstenfeuer von Elektromotoren im Diagramm erkannt und deren Beitrag zur Linienform durch dem Fachmann bekannte Software kompensiert werden.

Das erfindungsgemäße Verfahren lässt sich vorteilhaft auch dann einsetzen, wenn sich der magnetische Körper bereits im Magen befindet und dort zerfällt. Der magnetische Körper kann auch im Darm oder im Colon zerfallen. In diesen Fällen wird eine digitale Filterung in dem Bereich 0,1 bis 1 mHz bevorzugt. Soll der Schluckvorgang erfasst werden, ist ein Tiefpassfilter mit einer Grenzfrequenzbereich von 1 mHz - 0,99 Hz bevorzugt. Des Weiteren kann es vorteilhaft sein, die Auswahl der Filter und/oder Grenzfrequenzen dem geometrischen Aufbau des magnetischen Körpers, insbesondere der oralen Darreichungsform anzupassen. Der Zeitraum, in welchem sich eine orale Darreichungsform, zum Beispiel eine Kapsel zersetzt, liegt im Bereich von 0,5 - 30 min, bevorzugt im Bereich von 0,5 - 20 min, weiterhin bevorzugt im Bereich von 0,5 - 5 min. Sollen derart lang andauernde Vorgänge im menschlichen Körper gemessen werden, können die Signale bevorzugt "exponentiell geglättet" werden. Die mathematische Vorgehensweise hierfür ist dem Fachmann bekannt. Bevorzugte Glättungskonstanten $\alpha$ sind im Bereich von 0,10 bis 0,40, besonders bevorzugt liegt $\alpha$ bei ungefähr oder gleich 0,25.

Der magnetische Körper der Darreichungsform der erfindungsgemäßen Vorrichtung weist Untereinheiten auf, die Schichten, Phasen, und/oder Domänen sein können. Diejenige Untereinheit, die den magnetischen Fluss erzeugt, weist inerte, kristalline Teilchen auf, die Partikel, verglaste und/oder umhüllte Mikro- und/oder Minimagnete sein können. Vorzugsweise haben die Mikro- und/oder Minimagnete die Form von Zylindern, Schalen, und/oder Kugeln.

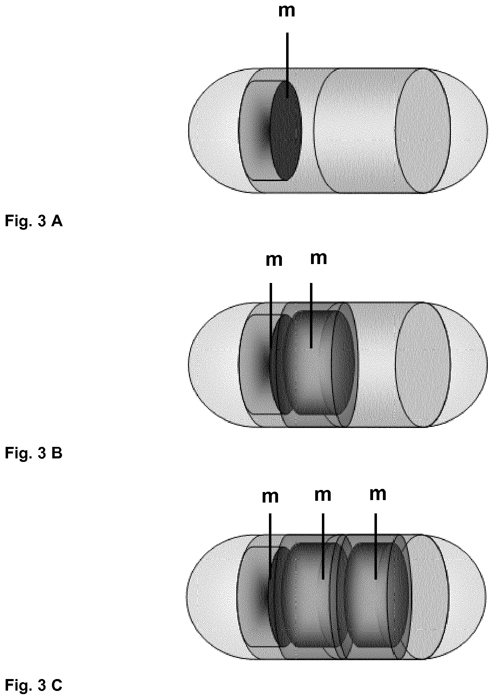

Bevorzugte Abmessungen der Mikro- bzw. Minimagnete sind von 0,1 bis 1 $\mu$m, von 1 bis 10 $\mu$m, von 10 bis 100 $\mu$m, von 100 $\mu$m bis 1 mm, und/oder von 1 mm bis 10 mm. Die Mikro- bzw. Minimagnete weisen magnetische Teilchen auf, vorzugsweise solche aus Magnetit und/oder einem magnetischen Material, welches der menschliche Organismus nicht verstoffwechselt. Des Weiteren können die magnetischen Teilchen mikrostrukturierte Polymerverbünde, und/oder teilkristalline, polymorphe, gesinterte, pulverförmige, oder Kombinationen davon aufweisen. Die magnetischen Teilchen können auch weitere, kommerziell gängige Komponenten aufweisen, vorzugsweise von diesen umhüllt sein, zum Beispiel von Dextranpartikeln, oder von anderen Komponenten für eine molekulare Umhüllung, zum Beispiel von Cyclodextrinen, oder von Komponenten, die durch Granulations- oder Pelletierverfahren erhalten sind. Indem die Mikro- bzw. Minimagnete durch diese verkapselt oder umhüllt sind, ist die systemische Aufnahme der Mikro- bzw. Minimagnete inhärent behindert. Vorzugsweise wird durch diese die Zersetzung der Mikro- bzw. Minimagnete durch die Magensäure verlangsamt und/oder der Beginn der Zersetzung verzögert. Mit dem fortschreitenden Zerfall wiederum schwächt derjenige magnetische Fluss bis zum Verschwinden ab, der mit dem erfindungsgemäßen Detektorsystem gemäß dem erfindungsgemäßen Verfahren erfasst wird. Die **Figuren 3 A - C** zeigen bevorzugte Ausführungsformen des magnetischen Körpers, und zwar in Form einer Kapsel, die jeweils mit einem (**Fig. 3 A**), zwei (**Fig. 3 B**), oder drei (**Fig. 3 C**) Minimagneten (m) ausgestattet ist.

Der magnetische Körper wird vorzugsweise mittels dem Fachmann bekannter galenischer Verfahren für die Herstellung oraler Darreichungsformen hergestellt, zum Beispiel mittels GMP-fähiger Herstellungsverfahren, vorzugsweise für die Herstellung von *Granules* mittels sogenanntem *high shear mixer*, oder in einem Wirbelschichtgranulator, mittels *roller compactor*, einem Extruder, Spheronisator, oder einem *hotmelt*-Prozess. Weiterhin bevozugt ist die Herstellung von sogenannten *Pellets* mittels dem Fachmann bekannnter *pelletization*, Extrusion und *spheronization*, *rotargranulation*, oder *powder layering.* Weiterhin können magnetische Körper in Form von Mikrotabletten aus teilkristallinem, verpresstem, verkapseltem, und/oder tablettiertem Material hergestellt werden, indem diese aus Pulver und polymorphen Substanzen kompaktiert werden. Orale Darreichungsformen können weiterhin in Form dem Fachmann bekannter Briefchen, den sogenannten *Sachets*, hergestellt werden.

Auch sind komplexere Formen magnetischer Körper denkbar, in denen zum Beispiel der magnetische Anteil die Form eines oder mehrerer Filme aufweist. Magnetische Körper des erfindungsgemäßen Detektorsystems können in jeder beliebigen Kombination der oben genannten Verfahren erhalten werden. Diese können weiterhin Vielteilchen-Systeme, Mehrschichtsysteme, core-shell-Systeme, und/oder Co-Block-Systeme sein.

Die orale Darreichungsform kann jede beliebige Form aufweist, die zumindest eine magnetische Phase aufweist, wobei unter *"magnetischer Phase"* ein im magnetischen Körper räumlich abgegrenzter Körper verstanden wird, der einen magnetischen Fluss verursacht. Dieser wird gemäß dem erfindungsgemäßen Verfahren erfasst. Die orale Darreichungsform wird nach Einnahme in den menschlichen Körper in einer definierten Zeitdauer zersetzt. Sind zum Beispiel zwei, drei, vier oder fünf magnetische Phasen enthalten, können diese Zeitdauern unterschiedlich lang, bevorzugt paarweise unterschiedlich lang sein. Die unterschiedliche Länge der Zeitdauern kann zum Beispiel dadurch erreicht werden, dass das magnetische Material in einen Polymerfilm gehüllt wird.

Ist die orale Darreichungsform eine Kapsel, kann beispielsweise eine Hälfte der Kapsel mit dem magnetischen Material befüllt sein. Weiterhin kann das magnetische Material zu einer Tablette gepresst in der Kapsel verbracht sein. Die magnetische Phase kann vorzugsweise von einer Hülle umgeben sein, die resistent gegen Magensäure ist und die mit der Hülle der oralen Darreichungsform zusammenfällt oder von dieser verschieden ist. Die Funktionen solcher langsam zerfallenden Hüllen, auch *"Überzüge"* oder *"Matrixstrukturen"* genannt, ist dem Fachmann bekannt. Mit dem Beginn des Zerfalls der Hüllen setzt selbstredend auch der Zerfall des magnetischen Materials dann ein, sobald dieses mit dem Medium in Kontakt kommt, das den Zerfall der Hülle bewirkt bzw. bewirkt hat. Mit dem Zerfall des magnetischen Materials geht die den magnetischen Fluss verursachende kollektive Ordnung der Elektronenspins verloren, und mit dem Verlöschen der kollektiven magnetischen Ordnung schwächt der magnetische Fluss bis zur Unmessbarkeit oder seinem Verschwinden ab.

Das Material einer langsam zerfallenden Hülle oder Verkapselung kann ausgewählt sein aus filmbildenden Polymeren. Diese können zum Beispiel Copolymere aus Methylmethacrylat und Ethylacrylat, Copolymere aus Methylmethacrylat und Ethylacrylat und Methacrylsäure, Copolymere aus Methylmethacrylat und Methylmethacylat und Methacrylsäure und Copolymere aus Methylmethacrylat, Ethylacrylat und Trimethylammoniumethylmethacrylat sein. Insbesondere geeignet sind Copolymere vom Typ EUDRAGIT® E100, EUDRAGIT® E PO, EUDRAGIT® L100, EUDRAGIT® L100-55, EUDRAGIT® S, EUDRAGIT® FS, EUDRAGIT® RS oder EUDRAGIT® RL. EUDRAGIT® NE oder EUDRAGIT® NM.

Geeignet sind weiterhin Polyvinylpyrolidone (PVP), Polyvinylalkohole, Polyvinylalkohol-Polyethylenglycol-Graft-Copolymer (Kollicoat®), Stärke und deren Derivate, Polyvinylacetatphtalat (PVAP, Coateric®), Polyvinylacetat (PVAc, Kollicoat), Vinylacetat-Vinylpyrolidon-Copolymer (Kollidon® VA64), Vinylacetat: Crotonsäure-Copolymere, Polyethylenglykole mit einem Molekulargewicht über 1000 (g/mol), Chitosan, ein (Meth)acrylatcopolymer, bestehend aus 20 - 40 Gew.-% Methylmethacrylat und 60 bis 80 Gew.-% Methacrylsäure, bekannt als ist EUDRAGIT® S, eine vernetzte und/oder unvernetzte Polyacrylsäure, als Smartseal® bekannte Fissurenversiegeler auf Compositbasis, Salz der Alginsäure und/oder ein Pektin, Cellulosen wie z. B. anionische Carboxymethylcellulose und deren Salze (CMC, Na-CMC, Ca-CMC, Blanose, Tylopur), Carboxymethylethylcellulose (CMEC, Duodcell®), Hydroxyethylcellulose (HEC, Klucel), Hydroxypropylcellulose (HPC), Hydroxypropylmethylcellulose (HPMC, Pharmacoat, Methocel, Sepifilm, Viscontran, Opadry), Hydroxymehylethylcellulose (HEMC), Ethylcellulose (EC, Ethocel®, Aquacoat®, Surelease®), Methylcellulose (MC, Viscontran, Tylopur, Methocel), Celluloseester, Celluloseglycolat, Celluloseacetatphtalat (CAP, Cellulosi acetas PhEur, Celluloseacetate-Phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF) ist oder eine Mischung der genannten Polymere ist.

Zusätzlich zu den filmbildenden Polymeren können in sich bekannter Weise weitere pharmazeutisch übliche Hilfsstoffe, die keine filmbildenden Polymere sind, als Formulierungshilfsmittel eingesetzt werden oder zusätzlich enthalten sein. Hier sind beispielhaft Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten. Weitere pharmazeutisch übliche Hilfsstoffe können in Mengen von 0,001 bis 30, bevorzugt 0,1 bis 10 Gew.-% bezogen auf das filmbildende Polymere vorliegen. Ebenfalls können dem Fachmann bekannte Hilfsstoffe für Tabletten, Kapseln, oder Arzneiformen eingesetzt werden.

Die orale Darreichungsform kann des Weiteren zumindest eine Schale und zumindest einen Kern aufweisen, welche die magnetischen Phasen sind und die im menschlichen Organismus der Reihe nach, von außen nach innen zersetzt werden, so dass der oder die Kerne den magnetischen Fluss am längsten aufrecht erhält oder erhalten.

Beispielsweise kann die Darreichungsform einen Kern in Form einer flachen Tablette aufweisen, wobei die flachen Seiten der Tablette die magnetische Phase sind, die mit einer weiteren Substanz fest verbunden, zum Beispiel chemisch oder mechanisch fixiert oder verschmolzen ist, und die dem menschlichen Organismus zugeführt werden soll. Diese Substanz kann zum Beispiel ein Wirkstoff, eine Arznei, oder allgemein eine biologisch aktive Substanz sein und an der Innenseite einer magnetischen Schale vorhanden sein. Die magnetischen Phasen der Tablette können verschieden dick oder in verschiedener Weise von einem weiteren Material ganz oder teilweise umhüllt sein, so dass die magnetischen Phasen binnen verschieden langer Zeitdauern zerfallen. Diese Zeitdauern können so gewählt sein, dass die magnetischen Phasen zerfallen, während die Darreichungsform im menschlichen Organismus transportiert wird, und somit jede magnetische Phase an einem anderen Ort im menschlichen Organismus zerfällt. Zum Beispiel kann eine Zeitdauer derart kurz gewählt sein, dass eine der magnetischen Phasen bereits während der Passage durch die Speiseröhre zerfällt.

In einer weiter bevorzugten Ausbildungsform kann die orale Darreichungsform zumindest drei Bestandteile aufweisen, von denen zumindest ein Bestandteil, vorzugsweise jedes Bestandteil eine magnetische Phase umschließt.

Die orale Darreichungsform kann des Weiteren zumindest drei Phasen aufweisen, von denen zumindest eine Phase eine biologisch aktive Substanz aufweisen kann, und die übrigen Phasen keine biologisch aktiven Substanzen enthalten, jedoch eine oder jeweils eine magnetische Phase. Solche Darreichungsformen lassen sich einfacher herstellen.

Die orale Darreichungsform kann ebenso bevorzugt eine magnetische Phase an oder auf ihrer äußeren Oberfläche aufweisen. Bei der Einnahme einer solchen Darreichungsform wird zunächst die magnetische Phase zersetzt. Danach erst kommen die übrigen Bestandteile der Darreichungsform mit dem menschlichen Organismus in Kontakt. Diese Ausführungsform hat nicht ausschließlich den Vorteil, dass die erfindungsgemäße Vorrichtung den genauen Zeitpunkt der Einnahme registriert. Der genaue Zeitpunkt der Einnahme kann zum Beispiel durch einen Peak in der zeitlichen Ableitung $\partial\Delta/\partial t$ der Meßsignalvektordifferenz erkannt werden, und/oder in einem plötzlichen Anstieg des Betrages von $\partial\Phi/\partial t$ über einen Wert hinaus, der zuvor festgelegt worden ist. Ein solcher Zeitpunkt ist im Rahmen der Erfindung gleichbedeutend mit der Detektion von sich verändernden Magnetfeldern und somit dem *Erkennen des Speiseröhren-Durchtritts.*

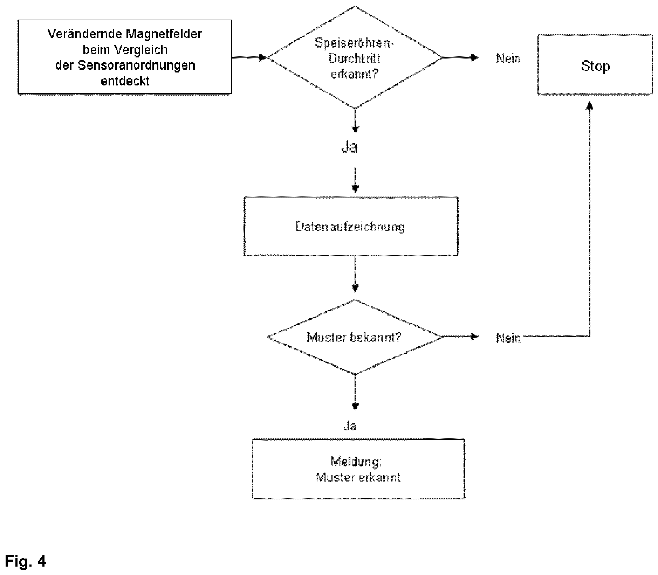

Bei der Aufzeichnung der Größe $\Delta$ und/oder $\Phi$ als Funktion der Zeit ist das Erkennen der Speiseröhre, bezeichnet mit, *"Speiseröhren-Durchtritt erkannt"* logisch positiv. Die Prozessierung dieses und des folgenden logischen Zustandes ist in der *Fig. 4* schematisch dargestellt.

Wenn andererseits der Zeitpunkt der Einnahme bekannt ist, wurde als weiterer Vorteil dieser Darreichungsform gefunden, dass verschiedene externe magnetische Flüsse oder Flussänderungen, die zu verschiedenen Zeitpunkten vorliegen und im Schritt (b) und/oder (c) nicht vollständig ausgeblendet oder herausgerechnet werden können, dennoch als störende Flüsse erkannt werden, indem die Linienform, die der magnetische Fluss der Darreichungsform nach dem Zeitpunkt der Einnahme in dem $\Delta$/t - oder $\Phi$/t - Diagramm jeweils erzeugt, als jeweiliges Charakteristikum für das Diagramm herangezogen wird. Dies kann bewerkstelligt werden, indem unmittelbar nach der erstmaligen Einnahme der Darreichungsform die Linienform während eines Zeitintervalls von 0 bis 10 s, vorzugsweise von 0 bis 5 s, tabelliert und/oder durch geeignete mathematische Funktionen approximiert wird. Unmittelbar nach jeder weiteren Einnahme zu jeweils bekannten Zeitpunkten kann die dann erfasste Linienform mit der tabellierten bzw. approximierten Linienform verglichen werden. Ein solcher Vergleich wird im Rahmen der Erfindung mit *"Datenaufzeichnung und Datenabgleich"* bezeichnet. Stimmt die erfasste Linienform in ihrer tabellierten und/oder approximierten Form mit der Linienform bei der ersten Einnahme der Darreichungsform überein, so ist dieser Befund, bezeichnet mit *"Muster bekannt"*, logisch positiv. Sind die logischen Werte *Speiseröhrendurchtritt erkannt* und *Muster bekannt* positiv, kann die Erfassung gemäß dem erfindungsgemäßen Verfahren vorgenommen werden, da nunmehr das vermittels der verändernden Magnetfelder gemessene *"Muster erkannt"* ist. Dann aber werden die weiteren Flussänderungen, die die Passage der Darreichungsform und deren Zersetzung im Organismus verursachen, trotz unterschiedlicher Umgebungen während verschiedener Zeitpunkte der Einnahme erfasst. Das ergibt den weiteren Vorteil einer Mobilität der erfindungsgemäßen Vorrichtung nahezu unabhängig von Ort oder Stärke externer magnetischer Flüsse, denn das erfindungsgemäße Verfahren unterscheidet nun sogar verschiedene, unbekannte externe Störeinflüsse. Ist wenigstens einer der beiden logischen Zustände negativ, kann die Erfassung vermieden, das erfindungsgemäße Detektorsystem ausgeschaltet, und/oder eine weitere Meldung generiert werden, die der Verwendung des Systems angepasst ist.

In einer weiteren Ausführung des erfindungsgemäßen Verfahrens kann die quantifizierte Bewertung dadurch vorgenommen werden, dass eine Zuordnung zumindest eines subjektiven Kriteriums, vorzugsweise Wohlbefinden und/oder körperliche Leistungsfähigkeit, zu alphanumerischen Zeichen, vorzugsweise einer Notenskala, durchgeführt und in das Logbuch eingetragen wird.

Das Logbuch registriert/speichert diese Zuordnung, zum Beispiel dem in Form einer Schulnote ausgedrückten Wohlbefinden, zusammen mit einer logischen Qualität gemäß *Fig. 4*, zum Beispiel "Muster bekannt". Es können auch weitere Daten miterfasst werden, z.B. Datum/Uhrzeit, und/oder besondere Vorkommnisse beim Erkennen des Speiseröhren-Durchtritts. Diese kann der menschliche Träger ebenfalls benoten und erfassen, z.B. besondere Stressoren, oder es kann, falls es sich ereignet hat, der mehrfache Abgleich einer erfassten Linienform mit der tabellierten Linienform infolge einer Störquelle erfasst werden. Somit hat die vorliegende Erfindung etliche Vorteile, indem viele Korrelationen erhalten werden können, die nur im Umfeld des menschlichen Trägers auftreten, und die vorzugsweise nur durch ihn wahrgenommen werden.

Selbst Störeinflüsse, die er nicht *bewusst* wahrnimmt, z.B. große magnetische Störfelder, die zu einem mehrfachen Abgleich der Linienformen führen, können dann beispielsweise mit dem Wohlbefinden und der Einnahme der oralen Darreichungsform korreliert werden.

Es kann des Weiteren vorteilhaft sein, die im Schritt (c) erhaltenen Werte für die Differenz und/oder für den Winkel Φ als Funktion der Zeit und die im Schritt (d) erfasste Bewertung als Funktion der Zeit aufzuzeichnen. Anhand dieser Aufzeichnung kann ausgesucht werden, welche Daten miteinander korreliert werden sollen. Beispielsweise können charakteristische Eigenheiten in den Δ/t- bzw. Φ/t-Diagrammen, falls vorhanden, mit dem subjektiven Kriterium als Funktion der Zeit korreliert werden. Solche Korrelationen sind bislang in keinem Datenmanagement Netzwerk zugänglich und können Aussagen über Verträglichkeit, Wirkung, angemessene Dosierung der oralen Darreichungsform geben. Außerdem sind nun Aussagen möglich, ob zwischen einer subjektiven Bewertung und irgendeiner der übrigen aufgezeichneten Daten überhaupt ein Zusammenhang besteht.

Die erfindungsgemäße Vorrichtung kann zur Erfassung geschluckter oraler Darreichungsformen und der Feststellung des oder der Zeitpunkte des Zerfalls des magnetischen, bevorzugt ferromagnetischen Anteils im Verdauungstrakt verwendet werden.

Der Vorteil besteht darin, dass zum Zeitpunkt des Zerfalls dieses Anteils, oder einen definierten Zeitraum davor, der magnetische Körper, allgemein die orale Darreichungsform ebenfalls zerfällt oder zerfallen sein muss, und somit darin eingeschlossene Substanzen freigesetzt sein müssen. *Die Detektion des Zerfalls kann so eine zeitliche Markierung dafür sein, wann zum Beispiel ein Wirkstoff einen bestimmten Teil des menschlichen Organismus erreicht. Zusammen mit dem Eintrag ins Logbuch hat die Verwendung den weiteren Vorteil, Aussagen über Wirksamkeit und/oder angemessene Anwendung der oralen Darreichungsform zu liefern.*

Vorzugsweise kann bei dieser Verwendung der Zerfall des magnetischen, bevorzugt ferromagnetischen Anteils im Magen, Dickdarm, Dünndarm, und/oder Colon festgestellt werden. Eine Option dieser Verwendung ist wie folgt.

Weist der magnetische Körper zumindest zwei magnetische Phasen auf, deren Zeitpunkte des Zerfalls so gewählt sind, dass diese magnetischen Phasen an unterschiedlichen Orten im menschlichen Organismus zerfallen, und ist außerdem mit jeder dieser magnetischen Phasen je eine Substanz fest verbunden, die von dem menschlichen Organismus aufgenommen wird und ein Lebensmittel, eine Nahrungsergänzung oder ein Genussmittel ist, und wird mit der Erfassung des jeweiligen Zerfalls außerdem eine Messung der Blutspiegel des oder der vom Organismus aufgenommenen Substanzen durchgeführt, dann kann zum Beispiel in klinischen Studien die Abgabe dieser Substanz oder Substanzen in vivo mit dem Verhalten des Metabolismus korreliert werden. Somit kann das Detektorsystem in allen Bereichen der Ernährung verwendet werden.

Bei der Verwendung werden die gemäß der Schritte (a) bis (d) erhaltenen Meßsignale und Einträge in zumindest einem Datenspeichergerät gespeichert, und die gespeicherten Daten und Einträge können vorzugsweise auf den Empfang eines Anforderungssignals hin an ein Empfangsgerät übermittelt werden.

Die Vorrichtung kann vorzugsweise die Signale über ein handelübliches Smartphone, Mobilphone, PDA übertragen, wobei durch einen weiteren Algorithmus an Bord dieses Kleincomputers eine Aufbereitung der Signale erfolgen kann. Ein Beispiel für eine solche Aufbereitung kann Datenreduktion, Verschlüsselung, und/oder Verrechnung mit persönlichen Daten des Trägers sein. Die von der erfindungsgemäßen Vorrichtung erhaltenen Signale können auf kabelgebundenem Wege, zum Beispiel vorübergehend mittels einer Steckverbindung, und/oder drahtlos übertragen werden, beispielsweise über Sensorknoten, Rechner, oder mittels Bluetooth®-Technologie auf ein mobiles Telefon. Nutzt man diese Technologie, kann der Aufwand für die Portierung der Software auf den digitalen Signalprozessor (DSP) eingespart und auch die Verarbeitungszeit verkürzt werden.

Das Datenspeichergerät kann ein Datalogger mit Sender sein, der zum Beispiel in Bluetooth®-Technologie ausgeführt sein kann. Ebenso ist denkbar, die erfindungsgemäße Vorrichtung mit einem Datalogger mit Sender, oder auch mit einem "Radio-Frequency Identification Device" (RFID) auszustatten. Mittels eines solchen Schaltkreises können bevorzugt einfach strukturierte Informationen gesendet und empfangen werden, beispielsweise solche gesendet werden, die mit einem besonderen Ereignis, z.B. einem Notfall verknüpft werden können. Diese Informationen können vorzugsweise aus den Meßsignalen abgeleitet werden, z.B. bei einem Missbrauch, Fehlverabreichung, zu häufiger oder zu seltener Dosierung, Unter- oder Überdosierung der oralen Darreichungsform, Energienotstand im oder Versagen der Vorrichtung. Auch können Systeme kombiniert werden, die bereits in der Medikation Anwendung finden, wie z.B. implantierte Schmerzmittelpumpen oder externe Perfusoren, die eine kontrollierte Injektion von Medikamenten steuern und wobei unter Umständen eine Kombination mit weiteren Medikamenten vermieden werden sollte.

Das Empfangsgerät kann jedes dem Fachmann bekannte Empfangsgerät sein, das von einem öffentlichen oder nicht-öffentlichen Server, Rechner und/oder Netzwerk unterstützt wird. Die empfangenen Daten können über ein Netzwerk aus Mobilfunkgeräten, Rechnern, Workstations, Kleincomputern, oder jedem sonstigen Rechner oder Server verarbeitet werden, das diese Daten besonders bevorzugt zum Zwecke ärztlicher Betreuung aufbereitet und/oder speichert. Es kann weiterhin vorteilhaft sein, die erfindungsgemäße Vorrichtung in einem öffentlichen oder nicht-öffentlichen Datenmanagement Netzwerk zu verwenden, ebenso bevorzugt beim Datenmanagement oder in einem Datenmanagement Netzwerk im Rahmen einer Therapie und/oder Diagnostik.

Das Datenmanagement Netzwerk kann von Experten abgerufen oder benutzt werden. Wird beispielsweise ein Notfall signalisiert, kann über ein automatisiertes System, z.B. über ein *"computerized physician order entry system"* (CPOE), ein Experte angefordert werden, zum Beispiel ein Notarzt. Der Experte korreliert die vom Datenmanagement Netzwerk gesammelten Daten, um den Ort und Zeitpunkt des Ereignisses, z.B. des Notfalls zu ermitteln, sowie um geeignete Maßnahmen zu ergreifen.

Die von der erfindungsgemäßen Vorrichtung erhaltenen und gegebenenfalls gesendeten Signale können prozessiert, codiert und/oder gepackt in das Datenmanagement Netzwerk übertragen werden. Die in dieses Datennetz übertragenen Daten können auf kommerziellem Wege mittels Telefonanruf abgerufen werden. Die übermittelten Daten können mittelbar oder unmittelbar, in Echtzeit und/oder gespeichert den zeitaufgelösten Zerfall des magnetischen Körpers protokollieren, bestätigen, oder dem Fachmann bekannter Weise weitere Eingabeaufforderungen auslösen.

Die erfindungsgemäße Vorrichtung kann im Rahmen von Behandlungen, Untersuchungen, Diagnosen, sowie bei der Erforschung neuer Therapien und Diagnosen, und im Rahmen der Verknüpfung der medizintechnischen Systeme verwendet werden.

Ebenso kann die erfindungsgemäße Vorrichtung bei der Durchführung und Kontrolle von gastrointestinaler Wirkstoffdosierung, insbesondere in soliden oder solid-flüssig-Kombinationspräparaten, verwendet werden.

Des Weiteren kann es vorteilhaft sein, diese Vorrichtung für Hochdurchsatz Tests zu verwenden. Mit Hilfe solcher Tests kann die Integrität der magnetischen Schichten, Phasen, und/oder Domänen geprüft, sowie das zeitliche Verhalten bei deren Auflösung im menschlichen Organismus bestimmt werden.

### Zusammenfassung

Die Erfindung betrifft eine Vorrichtung gemäß Anspruch 1 mit einem Sensorarrangement, das magnetische oder magnetisierte orale Darreichungsformen nach der oralen Einnahme detektieren kann, des Weiteren deren Auflösung über die Abnahme oder das Verschwinden des magnetischen Feldes der oralen Darreichungsform verfolgt, und mit einer Logbuchfunktion, die eine subjektive Bewertung vonseiten des menschlichen Trägers des Sensorarrangements bei oder nach der oralen Einnahme festhält.

### Patentansprüche

1. Vorrichtung, umfassend ein Detektorsystem zur Erfassung ein oder mehrerer durch den Menschen geschluckter magnetischer Körper im menschlichen Organismus, wobei jeder magnetische Körper mit je einer Substanz fest verbunden ist, die vom menschlichen Organismus aufgenommen wird, wobei die Substanz ein Lebensmittel, Nahrungsergänzung oder Genußmittel ist,

   wobei das Detektorsystem zumindest zwei Sensoranordnungen mit einem Gerät zur Aufzeichnung der von jeder Sensoranordnung gemessenen magnetischen Flussdichte aufweist, wobei jede Sensoranordnung von der oder den übrigen Sensoranordnungen einen Abstand von 0,5 bis 50 cm aufweist, und zumindest zwei Sensoranordnungen in einem Winkel von 0 bis 45 ° gegeneinander verkippt sind,

   und ein Logbuch zur Erfassung einer quantifizierten Bewertung vor, während und/oder nach der Erfassung des oder der magnetischen Körper

   aufweist, wobei

   die Sensoranordnungen in zumindest einem Gurt, der Kleidung, und/oder Schmuckgegenstand oder - gegenständen, in einem Armband, in einer Armbanduhr integriert, oder mittels Saugnapf oder Befestigungshilfe direkt am Körper fixiert sind, und das Logbuch am Körper mitgeführt ist,

   **dadurch gekennzeichnet, dass**

   jede Sensoranordnung einen, zwei, oder drei anisotrope Magnetwiderstandssensoren (AMR-Sensor) aufweist, deren Achsen der leichten Magnetisierung in paarweise unterschiedliche Richtungen weisen, und

   jede Sensoranordnung einen Vektor liefert, der sich aus den Meßsignalen der AMR-Sensoren zusammensetzt, wobei

die Vorrichtung dazu eingerichtet ist, die folgenden Schritte durchzuführen:

(a) zumindest einmaliges Aufschalten eines Set- und Reset-Impulses auf jeden anisotropen Magnetwiderstandssensor (AMR-Sensor),
(b) Verstärkung der Meßsignale jedes AMR-Sensors über eine geeignete Signalaufbereitung und über zumindest einen Tiefpassfilter,
(c) Bestimmung und Aufzeichnung der Differenz der Beträge der Vektoren der magnetischen Flussdichten jeder Sensoranordnung, und/oder Bestimmung und Aufzeichnung des Winkels Φ zwischen den Vektoren aus den Meßsignalen der AMR-Sensoren,
und
(d) zeitgleich mit einem der Schritte (a), (b) oder (c), oder nach Ablauf einer Zeit (T) im Anschluss an Schritt (c) zumindest einmalige Erfassung der quantifizierten Bewertung im Logbuch, die vom menschlichen Träger der Sensoranordnungenen vorgenommen wird.

2. Vorrichtung nach Anspruch 1, wobei der Gurt ein kombinierter Brust- und Schultergurt ist.

3. Vorrichtung nach Anspruch 1 oder 2, wobei der Abstand zwischen zwei Sensoranordnungen von 2 bis 6 cm gewählt ist.

**Claims**

1. Device, comprising a detector system for registering one or more magnetic bodies which have been swallowed by the person in the human organism,
wherein
each magnetic body is firmly connected to in each case a substance which is taken up by the human organism, wherein the substance is a foodstuff, food supplement or stimulant,
wherein the detector system comprises at least two sensor arrangements with an instrument for recording the magnetic flux density measured by each sensor arrangement,
wherein each sensor arrangement has a distance of 0.5 to 50 cm from the remaining sensor arrangement or sensor arrangements, and
at least two sensor arrangements are tilted at an angle of between 0 and 45° with respect to one another,
and has a log for registering a quantified evaluation before, during and/or after the registration of the magnetic body or bodies,
wherein the sensor arrangements are integrated in at least one strap, the clothing and/or an item or items of jewellery, in a bracelet, in a wristwatch, or affixed directly on the body by means of a suction cup or fastening aid,
and the log is also carried on the body,
**characterized in that**
each sensor arrangement has one, two or three anisotropic magnetoresistance sensors (AMR sensor), the axes of easy magnetization of which point in pair-wise different directions, and each sensor arrangement supplies a vector which is composed of the measured signals from the AMR sensors,
wherein the device is configured to carry out the following steps:

(a) applying a set and reset pulse, at least once, to each anisotropic magnetoresistance sensor (AMR sensor),
(b) amplifying the measured signals of each AMR sensor by means of suitable signal conditioning and by means of at least one low-pass filter,
(c) determining and recording the difference in the magnitudes of the vectors of the magnetic flux densities of each sensor arrangement, and/or determining and recording the angle Φ between the vectors from the measured signals of the AMR sensors, and,
(d) simultaneously with one of the steps (a), (b) or (c) or after a time (T) has elapsed following step (c), registering the quantified evaluation, at least once, in the log, which is undertaken by the human wearer of the sensor arrangements.

2. Device according to Claim 1, wherein the strap is a combined chest and shoulder strap.

3. Device according to Claim 1 or 2, wherein the distance between two sensor arrangments is chosen to be from 2 to 6 cm.

**Revendications**

1. Dispositif, comprenant un système de détecteur pour détecter un ou plusieurs corps magnétiques avalés par la personne dans le corps humain, dans lequel chaque corps magnétique est chaque fois attaché à une substance qui est assimilée par le corps humain, dans lequel la substance est un aliment, un complément alimentaire ou un stimulant, dans lequel le système de détecteur présente au moins deux agencements de capteur avec un appareil pour l'enregistrement de la densité de flux magnétique mesurée par chaque agencement de capteur, dans lequel chaque agencement de capteur présente une distance de 0,5 à 50 cm du ou des autres agencements de capteur, et au moins deux agencements de capteur sont inclinés d'un angle de 0 à 45° l'un par rapport à l'autre, et présente un journal pour consigner une évaluation quantifiée avant, pendant et/ou après la détection du ou des corps magnétique(s), dans lequel les agencements de capteur sont intégrés dans au moins une ceinture, des vêtements, et/ou un ou des bijoux, dans un bracelet, dans une montre-bracelet, ou sont fixés directement sur le corps au moyen d'une ventouse ou d'un accessoire de fixation, et le journal est emporté sur le corps, **caractérisé en ce que** chaque agencement de capteur présente un, deux ou trois capteur(s) magnétorésistif(s) anisotrope(s) (capteur AMR), dont les axes de la faible magnétisation pointent dans des directions différentes par paires, et chaque agencement de capteur fournit un vecteur, qui se compose des signaux de mesure des capteurs AMR, dans lequel le dispositif est conçu pour exécuter les étapes suivantes :

   (a) appliquer au moins une fois une impulsion de démarrage ou de réinitialisation à chaque capteur magnétorésistif anisotrope (capteur AMR),
   (b) amplifier les signaux de mesure de chaque capteur AMR au moyen d'un traitement de signal adéquat et au moyen d'au moins un filtre passe-bas,
   (c) déterminer et enregistrer la différence des valeurs des vecteurs des densités de flux magnétique de chaque agencement de capteur, et/ou déterminer et enregistrer l'angle $\phi$ entre les vecteurs composés des signaux de mesure des capteurs AMR, et
   (d) en même temps qu'une des étapes (a), (b) ou (c), ou après l'expiration d'un temps (T) à la suite de l'étape (c), consigner au moins une fois dans le journal l'évaluation quantifiée, qui est effectuée par le porteur humain des agencements de capteur.

2. Dispositif selon la revendication 1, dans lequel la ceinture est une ceinture pectorale et scapulaire combinée.

3. Dispositif selon une revendication 1 ou 2, dans lequel la distance entre deux agencements de capteur est choisie de 2 à 6 cm.

Gurt

Sensoranordnung
einkanalig

Sensoranordnung
dreikanalig

**Fig. 1**

**Fig. 2**

m

Fig. 3 A

m    m

Fig. 3 B

m    m    m

Fig. 3 C

Verändernde Magnetfelder
beim Vergleich
der Sensoranordnungen
entdeckt

Speiseröhren-
Durchtritt
erkannt?

Nein

Stop

Ja

Datenaufzeichnung

Muster bekannt?

Nein

Ja

Meldung:
Muster erkannt

**Fig. 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7698156 B2
- US 20100322859 A1
- DE 102011089334